# EUROPEAN PATENT SPECIFICATION

(11) **EP 0 486 425 B1**
(45) Date of publication and mention of the grant of the patent: **31.08.1994**
(21) Application number: 91810840.8
(22) Date of filing: 30.10.1991
(51) Int. Cl.: A23L 1/29, A23L 1/09, A23L 1/305

(54) **High acid system nutritional formulations**
Hochsaures System enthaltende Nahrungsformulierungen
Formules nitritives comprenant un système fortement acide

(30) Priority: 01.11.1990 US 608072
(43) Date of publication of application: 20.05.1992
(73) Proprietor: SANDOZ NUTRITION LTD., 3001 Bern (CH)
(72) Inventor: Kvamme, Candis D., Brooklyn Park, Minnesota 55445 (US)
(74) Representative: Smolders, Walter

(56) References cited:
- EP-A- 0 246 747
- EP-A- 0 265 772
- EP-A- 0 371 659
- WO-A-91/09538
- FR-A- 2 623 394
- US-A- 4 497 800
- US-A- 5 021 245

## Description

This invention relates to improved liquid nutritional formulations. More particularly, it relates to improved liquid oral nutritional formulations.

There are numerous oral nutritional formulations on the market. Currently most dairy based products are low acid (high pH) and typically vanilla, chocolate or strawberry flavored.

Most known oral nutritional formulations having a low pH, contain as dietary nitrogen source free amino acids and small peptides (see for example USP 4 497 800). Formulations comprising primarily free amino acids as a dietary nitrogen source have certain disadvantages, such as the high osmolality and the poor taste of such products.

The European Patent Application 0 246 747 relates to an enteral diet product with a pH value lower than 4.5 and comprising as dietary nitrogen compound proteins, more specifically protein hydrolysates, which are for at least 50 % of vegetable origin.

The not prior published international application WO 91/09538 discloses a nutritionally complete enteral product wherein the fat content on an energy basis is between 1 and 6 %, the pH value is below or equal to 4.5 and the osmolality is above 360 milli-osmol. Though it is stated in the latter application that all nutritionally compatible protein sources can be used, for instance whey protein and protein hydrolyzates, the disclosed examples contain soy protein hydrolysate.

The present invention provides improved dairy based liquid, oral nutritional formulations which have a juice-like consistency and flavor.

The formulation according to the invention comprise based on the total formulation calories from 40 to 90 % of calories as carbohydrates, from 2 to 30 % of calories as protein, from 0 to 35 % of calories as fat and from 0 to 17 % calories as fiber, whereby the protein source is for at least 60 % by weight whey protein concentrate and the pH of the formulation is from 3.5 to 3.9.

The formulations of the invention may also comprise further additives, such as vitamins, minerals, trace elements, flavoring agents, sweetening agents and the like. The formulations may be carbonated or non-carbonated.

The carbohydrate of the formulation may be any nutritionally acceptable carbohydrate source or blend of carbohydrate sources providing the desired amount of calories.

Examples of suitable carbohydrate sources for use in the formulation of the invention, include sucrose, glucose, fructose, corn syrup solids and maltodextrin.

The preferred carbohydrate source is sucrose.

When using corn syrup solids or maltodextrin, it is preferred that they be used in combination with either sucrose, glucose or fructose, or combinations of sucrose, glucose or fructose with the corn syrup solids or maltodextrin content of the combination being less than abut 40 % by weight of the total combination. Also, combinations of corn syrup solids and maltodextrin may be used with either sucrose, glucose, fructose, or combinations of sucrose, glucose or fructose provided that the combination of corn syrup solids and maltodextrin is less than about 40 % by weight of the total combination. Concentrations of corn syrup solids, maltodextrin or combinations thereof, are maintained below 40 % by weight of the total carbohydrate source to minimize Maillard browning.

Artificial sweeteners e.g., saccharin and aspartame, may also be used to enhance the organoleptic quality of the formulations.

The preferred amount of carbohydrate in the formulation of the invention, is from about 60 % to 85 % of the total formulation calories.

Examples of suitable protein source for use in the formulation of the invention include whey protein concentrate (hereinafter designated whey) or combinations thereof with caseinate, soy protein and/or egg whites. Whey is preferred because of its good flavor, and solubility at low pH. However, caseinate, soy protein and egg white may be used in combination with the whey, provided at least 60 % of the dietary nitrogen source is whey. At concentrations above 40 %, caseinate and soy protein tend to precipitate and impart a poor taste to the formulations, whereas egg whites at concentrations above 40 % tend to gel at processing temperatures.

The preferred amount of protein in the formulation of the invention, is from about 5-25 % of the total formulation calories.

The fat source of the formulations may be any nutritionally acceptable fat source or blend of fat sources providing the desired amount of fat calories. Preferably the fat source should be high in monounsaturated fatty acids. Examples of suitable fat sources include vegetable oils, e.g., high oleic acid vegetable oils such as sunflower oil, canola oil, and olive oil; safflower oil, cottonseed oil, corn oil or soybean oil, and medium chain triglycerides e.g., C6-C12 triglycerides. High oleic acid sunflower oil is preferred.

Marine oils and butter fat may also be used.

The fat source may preferably be from about 0 to 25 % of the total formulation calories.

Where it is not necessary to add fibers in formulations without fat, it may be advantageous to add fibers in formulations containing a fat source. Fat containing formulations according to the invention contain conveniently from 10 to 25 %, e.g. 20 % fat on an energy basis.

The fiber source may be insoluble fibers such as soy polysaccharide or soluble fibers such as guar gum, pectin, gum arabic or combinations thereof. Guar gum and pectin are preferred, in particular when they are in hydrolysed form. The fiber may preferably be from about 0-5 % of the total formulation calories.

Where the formulation of the invention comprises vitamins, it may comprise the nationally recommended allowances, conveniently, in 1 l of formulation.

Where the formulation of the invention comprises minerals, the mineral content should not be beyond a level that would cause gellation of the protein. Such level can be easily determined by standard tests.

It has been found that during heat processing and storage time, a high ion concentration , especially sodium ion and potassium ion, causes gellation of the protein of the formulations. To overcome this problem, the potassium content of the formulations is conveniently limited to about 0 to 10 mg per 100 ml (0 to 23.7 mg per 8 oz); and the sodium content of the formulations is conveniently limited to about 0 to 30 mg per 100 ml (0 to 71.1 per 8 oz). It will be understood that the sodium and potassium ions are supplied naturally (without added salts) by adjusting the combination of carbohydrates and protein. High amounts of macronutrients such as calcium or magnesium may also impair the palatability of the product. In general, formulations of satisfactory quality in terms of stability and palatability will be obtained with formulations comprising the nationally recommended daily allowances of minerals in one liter formulation. Examples of suitable formulations of the invention provide from 0.7 to 2.0 calories, e.g. 1.0 calorie per ml formulation. The formulation is conveniently ingested in the form of servings of from 150 to 300 ml.

The presence of reducing sugars and protein (which contain alpha amino acid groups) makes the formulations susceptible to nonenzymatic browning, which causes an undesirable color and unpleasant flavor. This browning effect may be reduced by maintaining the formulations at a low pH of 3.5-3.9. Further reduction in nonenzymatic browning without pH change may be achieved by the addition of effective amounts of nutritionally acceptable inhibitors of the Maillard reaction, such as cysteine or by the use of sucrose, a non-reducing sugar, or a combination of both.

Acids such as phosphoric acid, citric acid, malic acid, tartaric acid, fumaric acid, adipic acid, lactic acid, or combinations thereof, may be used for pH control. However, the combination of phosphoric acid and citric acid is preferred. A ratio of citric acid to phosphoric acid of 1 to 1.9 preferably 0.77 to 1.0 may be so used for pH control.

To inhibit non-enzymatic browning, cysteine may be added in free form from about 0.025 to 0.20 % on a weight/weight (w/w) basis, based on the total weight of the liquid formulation. The preferred amount is from about 0.04 % to 0.1 % (w/w).

Free amino acids may be added to the present formulations for nutritional benefits. Supplementation with free amino acids normally imparts a detrimental flavor to nutritional products. However, amino acids such as arginine, cysteine, isoleucine, leucine, methionine, valine or combinations thereof may be added to the present formulations without negative flavor impact due to the present low pH system. Preferred formulations comprise arginine as a supplement. Arginine is known to have valuable therapeutical properties; it does for example enhance wound healing and improve the immune response.

Where amino acids are added, they are preferably added in amounts of from about 0.2 to 7.0 %, more preferably of from 0.3 to 4 %, particularly of from 0.4 to 2 %, e.g. 1.0 % (w/w) basis, based on the total weight of the liquid formulation.

The following Tables I, II and III illustrate preferred nutritional formulations.

Table I shows the nutritional profile of formulations with and without fat. Table II lists the actual ingredients of the nutritional profile of Table I without fat. Table III lists the actual ingredients of the nutritional profile of Table I with fat.

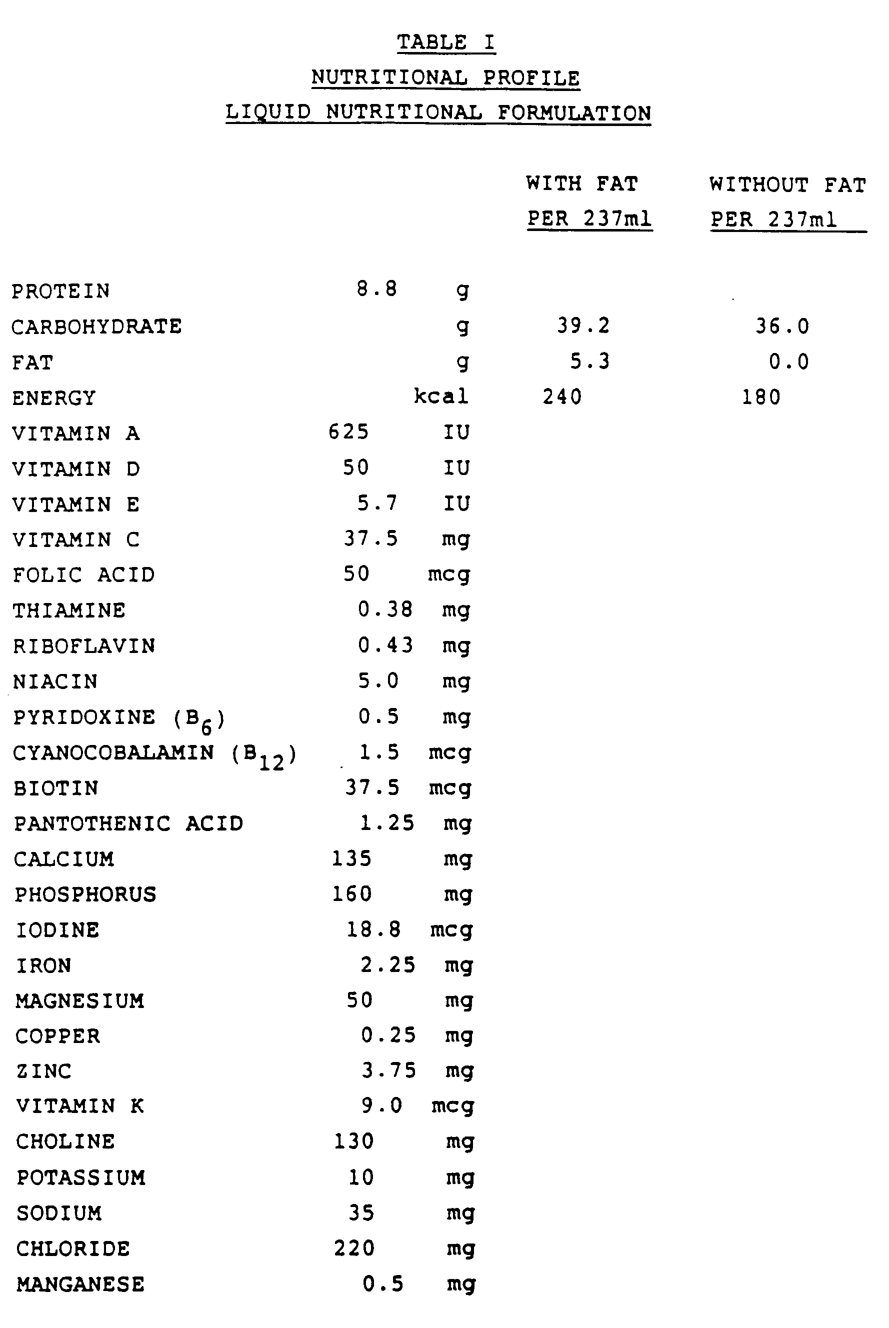

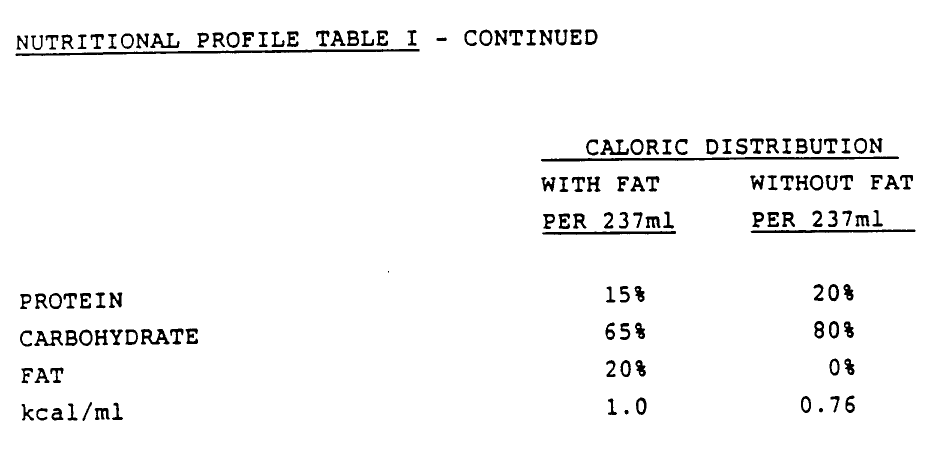

**TABLE II**

| ACTUAL INGREDIENTS LIQUID NUTRITIONAL FORMULATION WITHOUT FAT | | |
|---|---|---|
| Ingredients | GRAMS/237ml | % Formula |
| Deionized Water | 203.2 | 80.4 |
| Sucrose | 35.64 | 14.1 |
| Whey Protein Concentrate | 9.81 | 3.88 |
| Magnesium Gluconate | 1.01 | 0.400 |
| Phosphoric Acid (75%) | 0.784 | 0.310 |
| Citric Acid | 0.455 | 0.180 |
| Calcium Chloride | 0.480 | 0.190 |
| Corn Syrup Solids | 0.526 | 0.208 |
| Choline Bitartrate | 0.306 | 0.121 |
| Flavor/Color orange/yellow No. 6 | 0.265 | 0.105 |
| Ascorbic Acid | 0.095 | 0.038 |
| Cysteine | 0.1264 | 0.050 |
| Vitamin E Acetate | 0.0296 | 0.0117 |
| Zinc Sulfate | 0.01360 | 0.00538 |
| Ferrous Sulfate | 0.00841 | 0.00333 |
| Niacinamide | 0.00654 | 0.00259 |
| Vitamin A Palmitate | 0.00500 | 0.00198 |
| d-Calcium Pantothenate | 0.003020 | 0.001195 |
| Cyanocobalamin | 0.002480 | 0.000981 |
| Copper Gluconate | 0.002230 | 0.000882 |
| Manganese Sulfate | 0.002120 | 0.000840 |
| Vitamin K₁ | 0.001650 | 0.000653 |
| Thiamine Hydrochloride 0.000350 | 0.000885 | |
| Pyridoxine Hydrochloride | 0.000880 | 0.000348 |
| Vitamin D₃ | 0.000800 | 0.000320 |
| Riboflavin | 0.000556 | 0.000220 |
| Folic Acid | 0.000074 | 0.000029 |
| Biotin | 0.000058 | 0.000023 |
| Potassium Iodide | 0.000032 | 0.000013 |
| | 252.8 gm | 100.% |

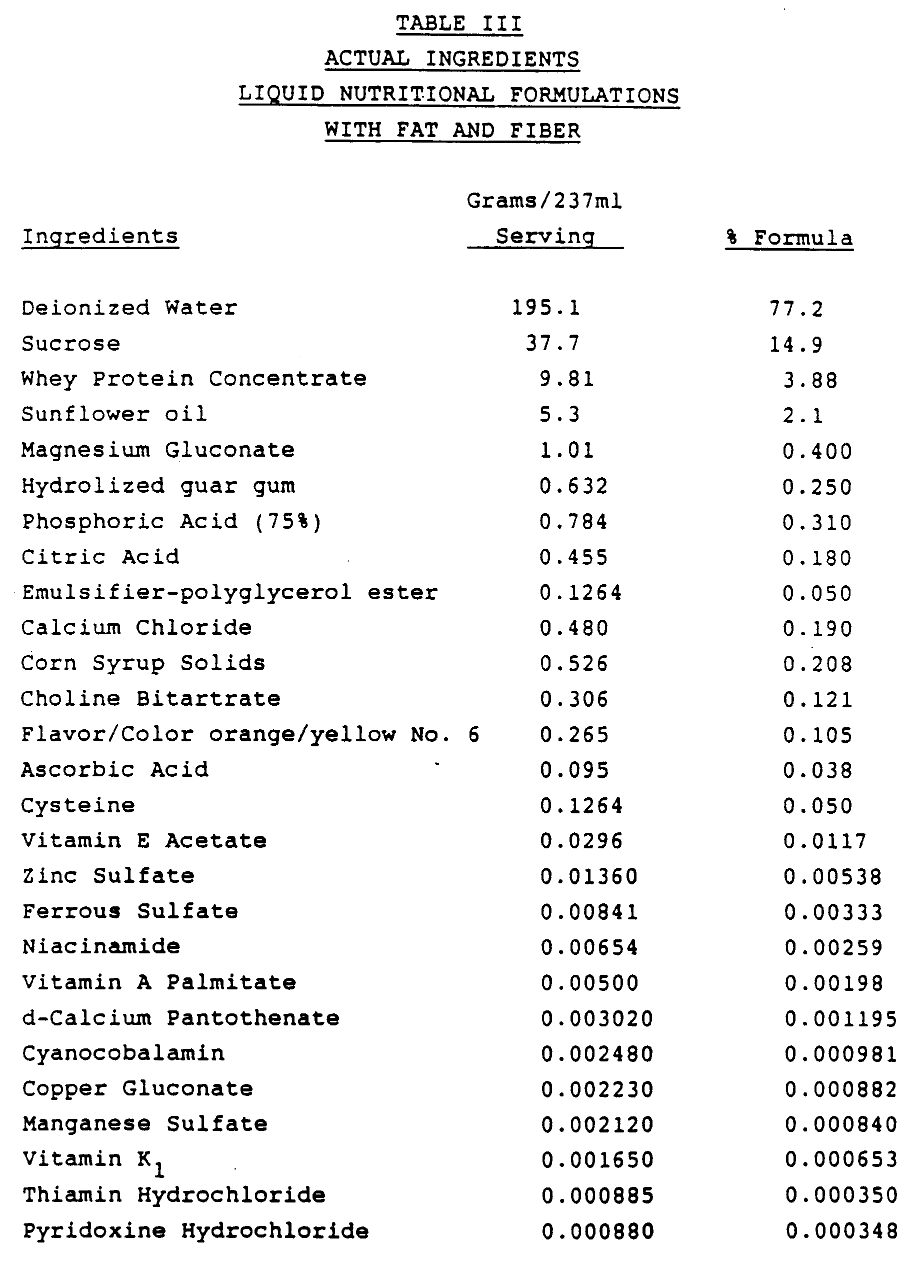

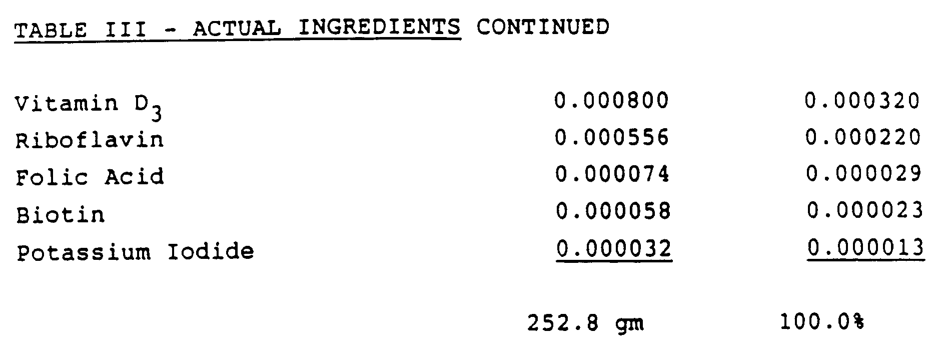

### EXAMPLE 1: Liquid Nutritional Formulation without Fat (3,624 kg)

140.6 kg of whey protein concentrate, and 511.0 kg of sucrose are added to 2,687.2 kg of deionized water in a 3,785 liter processing vessel. A solution of 11.2 kg of phosphoric acid and 6.5 kg of citric acid in 226.5 kg of deionized water are slowly added with agitation to the mixture in the processing vessel.

35.3 kg of a vitamin/mineral premix, and 1.8 kg of cysteine are, then added with agitation to the processing vessel, and allowed to mix thoroughly.

The formulation (21-26.7°C) from the processing vessel is pumped through a two-stage Gaulin homogenizer. The second stage pressure is 3,447 kPa, and the first stage pressure is 17,235 kPa. The product flows from the homogenizer to a plate heat exchanger where it is cooled to 4.4°C and then pumped to a 2nd 3,785 liter processing vessel. 3.8 kg of flavor and color orange/yellow No. 6 are added slowly with agitation to the 2nd vessel.

After 10 minutes of mixing, quality control samples are taken for moisture (80.0 %), pH (pH 3.8), specific gravity (1.067) and vitamin content (48 mgC/100 gms of solution).

The product is sterilized in a Cherry Burrell Unitherm thermo-processor at a minimum processing temperature of 95.6°C, for 4.33 seconds residence in the hold tube.

The product is then cooled to 71°C and aseptically homogenized in a two-stage homogenizer (pressure settings as above), and aseptically packaged (at 21.1°C) in 8 oz. (227 ml) Tetra Brik packages.

### EXAMPLE 2: Liquid Nutritional Formulation with Fat and Fiber (3,624 kg)

An emulsifier solution of 1.8 kg of polyglycerol ester in 45.3 kg of (82.2° C) deionized water is added to 2,412.7 kg of deionized water in a 3,785 liter processing vessel. 9.1 kg of hydrolysed guar gum, 140.6 kg of whey protein concentrate, and 540 kg of sucrose are added to the processing vessel and mixed with the emulsifier solution. 76.1 kg of high oleic sunflower oil is then added to the processing vessel and the ingredients mixed thoroughly. A solution of 11.2 kg of phosphoric acid and 6.5 kg of citric acid in 226.5 kg of deionized water are slowly added with agitation to the mixture in the processing vessel. The mixture is then heated to 71.1-73.9°C.

28.1 kg of a mineral premix, and 1.8 kg of cysteine are then added with agitation to the processing vessel, and allowed to mix thoroughly.

The formulation (71.1-73.9°C) from the processing vessel is pumped twice through a two-stage Gaulin homogenizer. The second stage pressure is 3,447 kPa, and the first stage pressure is 17,235 kPa. The product flows from the homogenizer to a plate heat exchanger where it is cooled to 4.4°C. The mixture is then pumped to a 2nd 3,785 liter processing vessel. 7.2 kg of vitamin premix in 113.3 kg of deionized water are added to the processing vessel. 3.8 kg of flavor and color orange/yellow No. 6 are then added slowly with agitation to the 2nd vessel.

After 10 minutes of mixing, quality control samples are taken for moisture (78 %), pH (pH 3.8), specific gravity (1.067) and vitamin C content (48 mgC/(100 gms of solution).

The product is sterilized in a Cherry Burrell Unitherm thermo-processor at a minimum processing temperature of 95.6°C, for 4.33 seconds residence in the hold tube.

The product is then cooled to 71.1°C and aseptically homogenized in a two-stage homogenizer (pressure settings as above), and aseptically packaged (at 21.1°C) in 8 oz. (227 ml) Tetra Brik packages.

## Claims

1. Liquid oral nutritional formulations comprising, based on the total formulation calories, from 40 to 90 % of the calories as carbohydrates, from 2 to 30 % of the calories as protein, from 0 to 35 % of the calories as fat and from 0 to 17 % of the calories as fiber, whereby the protein source is for at least 60 % by weight whey protein concentrate and the pH of the formulation is from 3.5 to 3.9.

2. The formulations of Claim 1, wherein the carbohydrates comprise from about 60 to 85 % of the total formulation calories.

3. The formulations of Claims 1 or 2, wherein the protein comprises from about 5 to 25 % of the total formulation calories.

4. The formulations of Claims 1 to 3, wherein the fat comprises from about 0 to 25 % of the total formulation calories.

5. The formulations of Claims 1 to 4, wherein the fiber comprises from about 0 to 5 % of the total formulation calories.

6. The formulations of Claims 1 to 5, wherein the carbohydrate source is selected from the group consisting of sucrose, corn syrup solids, glucose, fructose, maltodextrin, and combinations thereof.

7. The formulations of Claim 6, wherein at least 60 % by weight of the total carbohydrate combination is sucrose, glucose, fructose or a combination thereof.

8. The formulations of Claims 1 to 7 wherein the protein source is selected from the group consisting of whey protein or of combinations thereof with one or more of the dietary nitrogen sources caseinate, soy protein and egg whites.

9. The formulations of Claims 1 to 8, wherein the potassium content of the formulations is from about 0 to 10 mg per 100 ml of formulation, and the sodium content of the formulations is from about 0 to 30 mg per 100 ml of formulation.

10. The formulations of Claims 1 to 9, wherein the pH is controlled by acids selected from the group consisting of phosphoric acid, citric acid, tartaric acid, fumaric acid, adipic acid, lactic acid, and combinations thereof.

11. The formulations of Claim 10, wherein the acids are a combination of phosphoric acid and citric acid.

12. The formulation of Claims 1 to 11, having in addition a free amino acid selected from arginine, cysteine, methionine and combinations thereof.

13. The formulation of Claim 12, comprising free amino acids in the range of from 0.2 to 7.0 % by weight of the formulation.

14. The formulation of Claim 13, comprising free amino acids in the range of from 0.3 to 4.0 % by weight of the formulation.

## Patentansprüche

1. Flüssige orale Nahrungsmittelpräparate, umfassend auf Basis der Gesamtkalorien des Präparates 40 bis 90% der Kalorien als Kohlenhydrate, 2 bis 30% der Kalorien als Protein, 0 bis 35% der Kalorien als Fett und 0 bis 17% der Kalorien als Faser, wobei die Proteinquelle aus mindestens 60 Gew.-% Molkeproteinkonzentrat gebildet wird und der pH des Präparates 3,5 bis 3,9 ist.

2. Präparat nach Anspruch 1, worin die Kohlenhydrate etwa 60 bis 85% der Gesamtkalorien des Präparates ausmachen.

3. Präparat nach Anspruch 1 oder 2, worin das Protein etwa 5 bis 25% der Gesamtkalorien des Präparates bildet.

4. Präparat nach einem der Ansprüche 1 bis 3, worin das Fett etwa 0 bis 25% der Gesamtkalorien des Präparates bildet.

5. Präparat nach einem der Ansprüche 1 bis 4, worin die Faser etwa 0 bis 5% der Gesamtkalorien des Präparates bildet.

6. Präparat nach einem der Ansprüche 1 bis 5, worin die Kohlenhydratquelle ausgewählt ist aus der Gruppe bestehend aus Saccharose, Maissirupfeststoffen, Glucose, Fructose, Maltodextrin und Kombinationen davon.

7. Präparat nach Anspruch 6, worin mindestens 60 Gew.-% der Gesamtkohlenhydratkombination Saccharose, Glucose, Fructose oder eine Kombination davon ist.

8. Präparat nach einem der Ansprüche 1 bis 7, worin die Proteinquelle ausgewählt ist aus der Gruppe bestehend aus Molkeprotein oder Kombinationen davon mit einer oder mehreren der Nahrungsmittelstickstoffquellen Caseinat, Sojaprotein und Eiweiß.

9. Präparat nach einem der Ansprüche 1 bis 8, worin der Kaliumgehalt des Präparates etwa 0 bis 10 mg pro 100 ml Präparat ist und der Natriumgehalt des Präparates etwa 0 bis 30 mg pro 100 ml Präparat ist.

10. Präparat nach einem der Ansprüche 1 bis 9, worin der pH kontrolliert wird durch Säuren ausgewählt aus der Gruppe bestehend aus Phosphorsäure, Zitronensäure, Weinsäure, Fumarsäure, Adipinsäure, Milchsäure und Kombinationen davon.

11. Präparat nach Anspruch 10, worin die Säuren eine Kombination von Phosphorsäure und Zitronensäure sind.

12. Präparat nach einem der Ansprüche 1 bis 11, das zusätzlich eine freie Aminosäure ausgewählt aus Arginin, Cystein, Methionin und Kombinationen davon enthält.

13. Präparat nach Anspruch 12, das freie Aminosäuren im Bereich von 0,2 bis 7,0 Gew.-% des Präparates enthält.

14. Präparat nach Anspruch 13, das freie Aminosäuren im Bereich von 0,3 bis 4,0 Gew.-% des Präparats enthält.

## Revendications

1. Formulations nutritionnelles liquides pour la voie orale comprenant, par rapport aux calories totales de la formulation, de 40 à 90% de calories sous forme de glucides, de 2 à 30% de calories sous forme de protéines, de 0 à 35% de calories sous forme de graisses et de 0 à 17% de calories sous forme de fibres, la source de protéines étant constituée pour au moins 60% en poids de concentré de protéines de petit lait et le pH de la formulation étant compris entre 3,5 et 3,9.

2. Les formulations selon la revendication 1, dans lesquelles les glucides représentent de 60 à 85% du total des calories de la formulation.

3. Les formulations selon la revendication 1 ou 2, dans lesquelles les protéines représentent environ de 5 à 25% du total des calories de la formulation.

4. Les formulations selon les revendications 1 à 3, dans lesquelles les graisses représentent environ de 0 à 25% du total des calories de la formulation.

5. Les formulations selon les revendications 1 à 4, dans lesquelles les fibres représentent environ de 0 à 5% du total des calories de la formulation.

6. Les formulations selon les revendications 1 à 5, dans lesquelles la source de glucides est choisie dans le groupe constitué par le saccharose, les produits solides du sirop de maïs, le glucose, le fructose, la maltodextrine et leurs associations.

7. Les formulations selon la revendication 6, dans lesquelles au moins 60% en poids des glucides totaux est constitué par du saccharose, du glucose, du fructose ou d'une association de ces composés.

8. Les formulations selon les revendications 1 à 7, dans lesquelles la source de protéines est choisie dans le groupe constitué par les protéines de petit lait et ses associations avec une ou plusieurs des sources azotées diététiques que sont le caséinate, les protéines du soja et les blancs d'oeufs.

9. Les formulations selon les revendications 1 à 8, dans lesquelles la teneur en potassium des formulations est d'environ 0 à 10 mg pour 100 ml de formulation et la teneur en sodium des formulations est d'environ 0 à 30 mg pour 100 ml de formulation.

10. Les formulations selon les revendications 1 à 9, dans lesquelles le pH est contrôlé par des acides choisis dans le groupe constitué par l'acide phosphorique, l'acide citrique, l'acide tartrique, l'acide fumarique, l'acide adipique, l'acide lactique et les associations de ces composés.

11. Les formulations selon la revendication 10, dans lesquelles les acides sont une association d'acide phosphorique et d' acide citrique.

12. Les formulations selon les revendications 1 à 11, ayant en outre un amino-acide libre choisi parmi l'arginine, la cystéine, la méthionine et les associations de ces composés.

13. Les formulations selon la revendication 12, comprenant une teneur en amino-acides libres de 0,2 à 7,0% en poids de la formulation.

14. Les formulations selon la revendication 13, comprenant une teneur en amino-acides libres de 0,3 à 4,0% en poids de la formulation.
